# EUROPÄISCHE PATENTANMELDUNG

(11) **EP 4 732 823 A1**
(43) Veröffentlichungstag der Anmeldung: **29.04.2026**
(21) Anmeldenummer: 25210048.2
(22) Anmeldetag: 21.10.2025
(51) Int. Cl.: A61K 8/02, A61K 8/27, A61K 8/29, A61K 8/37, A61K 8/39, A61K 8/92, A61Q 17/04

(54) **KOSMETISCHE ZUBEREITUNG**

(30) Priorität: 22.10.2024 DE 102024130721
(71) Anmelder: Laverana GmbH & Co. KG, 30974 Wennigsen (DE)
(72) Erfinder: König, Oliver Thomas, 30974 Wennigsen (DE); Neuhoff, Henrike, 30974 Wennigsen (DE)
(74) Vertreter: Scholz, Volker

(57) **Zusammenfassung**

Die vorliegende Erfindung betrifft eine kosmetische Zubereitung, die mindestens einen mineralischen Lichtschutzfilter, Polyglyceryl-4-diisostearat/Polyhydroxystearat/Sebacat, Polyhydroxystearinsäure, Isostearinsäure, Polyglyceryl-2-oleat und Polyglyceryl-2-stearat umfasst; sowie die Verwendung der Zubereitung zum Schutz vor Sonnenlicht.

## Beschreibung

Die vorliegende Erfindung betrifft eine kosmetische Zubereitung, insbesondere zur Verwendung zum Schutz vor Sonnenlicht.

Insbesondere betrifft die Erfindung eine kosmetische Zubereitung, die den Anforderungen der aktuellen Naturkosmetik-Zertifizierungen entspricht.

Die Entwicklungen der letzten Jahre in der Kosmetikindustrie, aber auch in anderen Branchen, haben gezeigt, dass die Nachfrage nach zertifizierter Naturkosmetik mit Lichtschutz eine immer größere Bedeutung erlangt. Der Verbraucher hat seine Lebensgewohnheiten geändert und achtet auch beim Einkauf von kosmetischen Mitteln zunehmend auf "natürliche" und nachhaltige Aspekte. Dieser Trend wird immer stärker, und die Naturkosmetik ist bereits ein festes Segment in der Kosmetik geworden.

Dies wird unterstützt durch die zunehmend kritische Einstellung vieler Verbraucher zu der Verwendung synthetischer und petrochemischer Produkte in kosmetischen Zubereitungen.

Der Verbraucher hat heutzutage folgende Erwartungen an einen mineralischen Sonnenschutz:
**Gesundheitliche Aspekte:** Mineralische Lichtschutzfilter reduzieren das Risiko von Hautirritationen und allergischen Reaktionen im Vergleich zu chemischen Filtern, die in die Haut eindringen können. Verbraucher, insbesondere diejenigen mit empfindlicher Haut oder Allergien, erwarten daher, dass mineralische Lichtschutzfilter sanft zur Haut sind und keine Reizungen oder allergischen Reaktionen hervorrufen. Darüber erwarten sie einen effektiven UV-Schutz gegen UVA- und UVB-Strahlen.

**Vielseitigkeit:** Verbraucher schätzen es, wenn mineralische Lichtschutzprodukte für verschiedene Hauttypen und Aktivitäten geeignet sind, sei es für den täglichen Gebrauch, sportliche Aktivitäten oder den Strandurlaub.

**Sofortiger Schutz:** Da mineralische Filter physikalisch wirken, bieten sie sofortigen Schutz vor UV-Strahlen, während einige chemische Filter etwa 20 Minuten benötigen, um ihre volle Schutzwirkung zu entfalten. Verbraucher erwarten, dass dieser Schutz unmittelbar nach dem Auftragen einsetzt.

**Umweltfreundlichkeit:** Chemische UV-Filter können Korallenriffe und andere Meereslebewesen schädigen. Mineralische Filter sind in der Regel umweltfreundlicher und sicherer für Meeresökosysteme. Verbraucher erwarten, dass diese Produkte keine schädlichen Auswirkungen auf die Umwelt haben und bevorzugen daher umweltfreundliche Optionen.

**Natürliche Inhaltsstoffe:** Verbraucher, die Wert auf natürliche und biologische Produkte legen, bevorzugen oft mineralische Filter, da sie aus natürlichen Mineralien gewonnen werden und keine synthetischen Chemikalien enthalten. Sie erwarten, dass die Produkte frei von synthetischen Duftstoffen, Farbstoffen und Konservierungsmitteln sind.

**Ästhetik und Komfort:** Verbraucher erwarten, dass mineralische Sonnenschutzprodukte leicht aufzutragen sind, keine weißen Rückstände hinterlassen und sich angenehm auf der Haut anfühlen.

Daher ist es wünschenswert, kosmetische Zubereitungen ohne die Verwendung von derartigen synthetischen und petrochemischen Inhaltstoffen herzustellen. Solche Zubereitungen werden oft als Naturkosmetik bezeichnet.

Eine gesetzliche Definition des Begriffs "Naturkosmetik" gibt es weder national noch international. Es gibt jedoch formelle Kriterien, welche Anforderungen an derartige Produkte zu stellen sind. Werden diese Kriterien von einem Produkt, einer Produktgruppe oder einem Unternehmen erfüllt, so kann ein Zertifikat ausgestellt werden.

In der zertifizierten Naturkosmetik werden gemäß der NATRUE-Zertifizierung grundsätzlich drei verschiedene Rohstoffklassen unterschieden. Zum einen sind dies natürliche Rohstoffe, die nur durch physikalische Mittel (z.B. Kaltpressen) gewonnen werden. Die zweite Klasse sind naturnahe Rohstoffe. Diese beinhalten chemisch veränderte natürliche Rohstoffe, wobei die chemischen Reaktionen ein Vorbild in der Natur haben (z.B. Verseifung, Veresterung). Zur Sicherstellung der mikrobiellen Sicherheit des Produktes können auch bestimmte naturidentische d.h. vollsynthetische Konservierungsstoffe eingesetzt werden, die eine dritte Klasse an Naturkosmetikrohstoffen darstellen. Deren Gewinnung aus der Natur kann aufwendig sein. Naturidentische Rohstoffe sind synthetisch hergestellt, jedoch mit dem natürlich vorkommenden Rohstoff identisch.

Für den Erhalt des Zertifikates der Organisation NATRUE für die Naturkosmetik ist es etwa erforderlich, Zubereitungen zu erhalten, die aus bestimmten prozentualen Verhältnissen von natürlichen, naturnahen und naturidentischen Inhaltstoffen bestehen. Weiter dürfen die enthaltenen Inhaltsstoffe nur naturähnlich chemisch modifiziert werden, etwa durch bestimmte Veresterungen, die unter naturähnlichen Bedingungen durchgeführt werden. Diese Formulierungen sind daher entsprechend frei von synthetischen Duftstoffen, frei von synthetischen Farbstoffen, frei von Silikonen und Silikonderivaten, frei von mit ionisierender Strahlung bestrahlten Pflanzeninhaltstoffen, frei von genmodifizierten Inhaltstoffen, frei von künstlichen organischen UV-Filtern, frei von Paraffinen, frei von Mineralöl, frei von Mikrokunststoffkugeln, frei von Flüssigkunststoffen und frei von Parabenen und anderen nicht unter NATRUE zugelassenen synthetischen Konservierungsstoffen.

Dies gilt ebenso für andere Naturkosmetik-Zertifikate wie EcoCert Cosmos, BDIH Cosmos, etc. Bei diesen Zertifizierungen gibt es allerdings keine Vorgaben für die Verhältnisse von natürlichen, naturnahen und naturidentischen Rohstoffen. Hier sind Mindestmengen an natürlichen und natürlichen Inhaltstoffen aus biologischem Anbau vorgegeben.

Durch die im Vergleich zum Massenmarkt begrenzte Auswahl an verwendbaren Rohstoffen, die es erlauben, ein Naturkosmetik-Zertifikat zu erhalten, ist die Wahlfreiheit bei der Formulierung naturkosmetischer Zubereitungen eingeschränkt. So steht eine Vielzahl von Emulgatoren, Tensiden, Silikonen, Ölkomponenten, Konservierungsstoffen, Duftstoffen, Polymeren, mineralischen Lichtfiltern und Wirkstoffen zur Formulierung naturkosmetischer Zubereitungen nicht zur Verfügung.

Dadurch können Produkteigenschaften, wie sie von üblichen Kosmetikprodukten bekannt sind, nicht immer einfach erreicht werden. Dies kann die Lagerstabilität der Zubereitung, ihr Fließverhalten, etwa bei Entnahme des Produktes aus der Verpackung, ihre sensorischen Eigenschaften, etwa beim und/oder nach dem Verteilen auf der Haut sowie die erreichbare Konditionierung und Wirksamkeit auf und in der Haut oder ihrer Anhangsgebilde betreffen. Die wichtigste Herausforderung ist es daher einhergehend mit der Auswahl der entsprechenden Rohstoffe, dem Verbraucher wirksame, sichere und sensorisch ansprechende, hochwertige Produkte anbieten zu können. Ein wesentlicher Aspekt ist hier auch das sogenannte "Weißeln", hiermit sind weiße Rückstände beim Auftragen von natürlichen Produkten auf der Haut gemeint.

Insbesondere beim Lichtschutz sind in der zertifizierten Naturkosmetik nur zwei mineralische Filter, namentlich Titandioxid und Zinkoxid, erlaubt. Künstliche organische Filter sind nicht erlaubt, da diese nicht den zugelassenen Herstellverfahren entsprechen oder nicht natürlicher Weise in der Natur vorkommen. Da diese UV-Filter sogenannte Weißpigmente sind, besteht gerade bei der Formulierung von Zubereitungen mit Sonnenschutz die Herausforderung, eine Formulierung zu entwickeln, die dieses "Weißel"-Verhalten möglichst nicht zeigt und zusätzlich trotz der hohen Anzahl von Pigmenten eine angenehme Sensorik aufweist. Darüber besteht außerdem eine weitere Herausforderung darin, diese mineralischen Lichtschutzfilter so in einer kosmetischen Zubereitung zu dispergieren, dass sie nicht agglomerieren und stabil in der Formel dispergiert sind und eine gleichmäßige und effektive Lichtschutzwirkung gewährleistet wird.

Nach dem aktuellen Stand der Technik versucht man, diese Herausforderungen durch den Einsatz von Polyethylenglycol, Polyethylenglycolethern und Polyethylenglycolestern (bekannt als PEG-Derivate) als Emulgatoren zu bewältigen. Diese PEG-Derivate sind jedoch bei Verbrauchern oft unbeliebt, da ihre Verträglichkeit immer wieder zur Diskussion steht und sie die Penetration von Inhaltsstoffen in tiefere Hautschichten fördern können. Der Wunsch der Verbraucher nach PEG-freien Formulierungen ist hoch.

Zudem wird versucht, die Stabilitätsprobleme durch die Zugabe von Polymeren und Copolymeren der Acrylsäure (wie Carbomere, Acrylates/Alkylacrylates-Crosspolymer) oder Copolymeren des Vinylpyrrolidons zu lösen. Im Zuge der "Mikroplastik"-Debatte wird der Einsatz dieser Stoffe jedoch zunehmend kritisch betrachtet. Auch hier bleibt die Frage offen, ob der rückstandsfreie Abbau dieser Stoffe in der Natur und in Kläranlagen problematisch ist. Entscheidend ist auch hier der Wunsch der Verbraucher nach Sonnenschutzprodukten, die als "mikroplastikfrei" gelten. Darüber hinaus entsprechen alle diese Formulierungen nicht den strengen Vorgaben der zertifizierten Naturkosmetik.

CN105456051 B offenbart eine natürliche Feuchtigkeitscreme mit verschiedenen Bestandteilen, allerdings ohne Lichtschutzfilter.

EP3093004 A1 beschreibt eine kosmetische Zubereitung mit synthetischen UV-Filtern. Zertifizierte naturkosmetische Zubereitungen mit Lichtschutz werden nicht offenbart.

DE10260877A1 beschreibt kosmetische und/oder dermatologische Öl-in-Wasser-Emulsionen, enthaltend Zinkoxid und Fettsäureester der Phosphor- oder Schwefelsäure mit kosmetischen und/oder dermatologischen Wirk-, Hilfs- und Zusatzstoffen, die allerdings nicht für die Naturkosmetik geeignet sind und nachweislich nicht den erforderlichen hohen Lichtschutz von bis zu 50 erreichen.

EP4096794A1 beschreibt ein Sonnenschutzmittel mit einem Pigmentfiltersystem, allerdings für sprühbare Zusammensetzungen mit Polyglyceryl-3-diisostearat, was nicht für Sonnenschutzcremes/Lotions geeignet ist.

Es ist somit eine Aufgabe der vorliegenden Erfindung, eine kosmetische Zubereitung, insbesondere eine kosmetische Sonnenschutzzubereitung, bereitzustellen, die das "Weißel"-Verhalten minimiert, stabil ist, eine gleichmäßige und effektive Lichtschutzwirkung erzielt und/oder aktuelle Anforderungen an eine Naturkosmetik-Zertifizierung erfüllt.

### Zusammenfassung der Erfindung

Die Aufgabe wird gelöst durch eine kosmetische Zubereitung, die mindestens einen partikulären mineralischen Lichtschutzfilter, Polyglyceryl-4-diisostearat/Polyhydroxystearat/Sebacat, Polyhydroxystearinsäure, Isostearinsäure, Polyglyceryl-2-oleat und Polyglyceryl-2-stearat umfasst, sowie deren Verwendung zum Schutz vor Sonnenlicht.

Weitere Ausführungsformen ergeben sich aus den Unteransprüchen.

Die kosmetische Zubereitung der vorliegenden Erfindung ist eine zertifizierte Naturkosmetik-Zubereitung, die den menschlichen Körper vor Sonnenlicht schützt, akzeptable sensorische Eigenschaften ohne starkes Weißeln aufweist und die Haut nachweislich ausreichend schützt. Zudem zeigt sie eine hinreichend stabile Dispergierbarkeit der Lichtschutzpigmente, um Agglomerationen zu vermeiden, welche zu einem niedrigeren Lichtschutzfaktor (SPF), Instabilitäten und erneutem Weißelverhalten führen könnten.

Mineralische Partikel, wie ZnO- und TiO₂-Partikel, neigen dazu, sich zu agglomerieren und zu sedimentieren, was zu ungleichmäßiger UV-Schutzwirkung führen kann. Die erfindungsgemäße kosmetische Zubereitung ist eine sehr stabile Dispersion, bei der die Partikel in der Emulsion gleichmäßig verteilt bleiben.

Um eine konsistente UV-Absorption und -Reflexion zu gewährleisten, müssen die mineralischen Partikel, wie ZnO- und TiO₂-Partikel, gleichmäßig in der Emulsion verteilt sein, was mit der erfindungsgemäßen kosmetischen Zubereitung erreicht wird. Die Oberfläche der mineralischen Partikelkann so modifiziert werden, dass sie besser mit der Emulsionsmatrix kompatibel sind, um eine stabilere Dispersion und eine gleichmäßigere Lichtschutzwirkung zu ermöglichen.

Der pH-Wert und die Ionenstärke der Emulsion können so eingestellt sein, dass chemische Reaktionen, die die UV-Schutzwirkung beeinträchtigen könnten, vermieden werden.

Die mineralischen Partikel, wie ZnO und TiO₂, sind mit anderen Inhaltsstoffen der Emulsion kompatibel, um chemische Reaktionen oder physikalische Instabilitäten zu verhindern, die die Lichtschutzwirkung beeinträchtigen könnten.

Die Viskosität der Emulsion kann so eingestellt werden, dass die mineralischen Partikel in Schwebe gehalten werden können, ohne dass die Emulsion zu dickflüssig wird, um eine gleichmäßige UV-Schutzschicht auf der Haut zu gewährleisten.

Die Emulsion bleibt über die gesamte Nutzungsdauer stabil, um eine konstante und zuverlässige Lichtschutzwirkung zu bieten. Instabilitäten könnten die Schutzwirkung im Laufe der Zeit verringern.

Darüber hinaus erfüllt die erfindungsgemäße kosmetische Zubereitung den Lichtschutzfaktor nicht nur über die derzeitig aktuellen ISO-Normen ISO 24444 und ISO 24443, sondern auch über die neuen "ethischen" Methoden, der ISO /DIS 23698 (HDRS-Methode) und der ISO /DIS 23675 in vitro (double plate method). Dies stellt insbesondere für den mineralischen Lichtschutz eine Herausforderung dar, da in-vitro Transmissionsmessungen auf einer aufgerauten Polymethylmethacrylat - Platte (PMMA - Platte) gemacht werden und die Auftragungsmenge rein nach Gewicht und nicht nach Volumenbasierten Ansatz vorgesehen ist, welcher nachteilig für mineralische Filter ist.

Zusammengefasst optimiert die erfindungsgemäße kosmetische Zubereitung all diese Faktoren, um eine stabile, gleichmäßige und langanhaltende Lichtschutzwirkung durch die Kombination von mineralischen Partikeln, wie ZnO und / oder TiO₂, in Emulsionen zu erreichen und zugleich die Ansprüche an die Ästhethik (Weißelverhalten) und den sensorischen Erwartungen der Verbraucher gerecht zu werden.

### Detaillierte Beschreibung der Erfindung

Es wurde überraschend und für den Fachmann nicht vorhersehbar festgestellt, dass die kosmetische Zubereitung der vorliegenden Erfindung stabile Dispersionen ermöglicht, die sowohl in Wasser-in-Öl- als auch in Öl-in-Wasser-Emulsionen stabil eingearbeitet werden können, ohne Agglomerate oder Weißelverhalten zu erzeugen.

Der partikuläre mineralische Lichtschutzfilter kann ausgewählt sein aus der Gruppe bestehend aus partikulärem Titandioxid, partikulärem Zinkoxid und einer Mischung derselben.

Dabei kann Titandioxid und/oder Zinkoxid mit einer Primärpartikelgröße zwischen 2 und 200 nm, 5-200 nm oder 5-100 nm umfasst sein. Die Primärpartikelgröße kann nach auf dem Fachgebiet bekannten Verfahren bestimmt werden, z.B. mittels Lichtstreuung.

Der partikuläre mineralische Lichtschutzfilter kann in einer Menge von 0,1 bis 50 Gewichtsprozent, 0,2 bis 50 Gewichtsprozent, 0,2 bis 40 Gewichtsprozent, 1 bis 35 Gewichtsprozent oder 5 bis 35 Gewichtsprozent, bezogen auf das Gesamtgewicht der Zubereitung, in der kosmetischen Zubereitung enthalten sein.

Dabei sind unter anderem drei Varianten denkbar:
i) Zubereitungen mit Titandioxid. In diesem Fall können die Einsatzmengen für Titandioxid zwischen 0,1 und 25 Gewichtsprozent, jeweils bezogen auf das Gesamtgewicht der Zubereitung, liegen.
ii) Zubereitungen mit Zinkoxid. Hier können die Einsatzmengen für Zinkoxid ebenfalls 0,1 bis 25 Gewichtsprozent, bezogen auf das Gesamtgewicht der Zubereitung, betragen.
iii) Zubereitungen mit einer Kombination aus Titandioxid und Zinkoxid. In diesem Fall können die Einsatzmengen für Titandioxid 0,1 bis 25 Gewichtsprozent und für Zinkoxid 0,1 bis 25 Gewichtsprozent, jeweils bezogen auf das Gesamtgewicht der Zubereitung, betragen.

Die Zubereitung kann ausschließlich Titandioxid in der Kristallstruktur Rutil enthalten. Ein Titandioxid gilt dabei als ausschließlich in dieser Kristallstruktur vorliegend, wenn der Anteil anderer Kristallstrukturen insgesamt 1 Gewichtsprozent, bezogen auf die Gesamtmenge an Titandioxid, nicht überschreitet. Polyglyceryl-4-diisostearat/Polyhydroxystearat/Sebacat kann in der Zubereitung in einer Menge von 0,1 bis 10 Gewichtsprozent, 1 bis 5 Gewichtsprozent oder 1 bis 4 Gewichtsprozent, bezogen auf das Gesamtgewicht der Zubereitung, vorliegen.

Polyhydroxystearinsäure kann in der Zubereitung in einer Menge von 0,1 bis 10 Gewichtsprozent, 1 bis 5 Gewichtsprozent oder 1 bis 4 Gewichtsprozent, bezogen auf das Gesamtgewicht der Zubereitung, vorliegen.

Isostearinsäure kann in der Zubereitung in einer Menge von 0,05 bis 0,5 Gewichtsprozent, 0,1 bis 4 Gewichtsprozent oder 0,1 bis 2 Gewichtsprozent, bezogen auf das Gesamtgewicht der Zubereitung, vorliegen.

Polyglyceryl-2-oleat kann in der Zubereitung in einer Menge von 0,05 bis 2 Gewichtsprozent, 0,1 bis 3 Gewichtsprozent oder 0,1 bis 1 Gewichtsprozent, bezogen auf das Gesamtgewicht der Zubereitung, vorliegen.

Polyglyceryl-2-stearat kann in der Zubereitung in einer Menge von 0,02 bis 1,5 Gewichtsprozent, 0,03 bis 1 oder 0,04 bis 1 Gewichtsprozent, bezogen auf das Gesamtgewicht der Zubereitung, vorliegen.

Die Kombination der Inhaltsstoffe in diesen Einsatzkonzentrationen führt zu besonders stabilen Emulsionen und darüber hinaus zu einer gleichmäßigeren Pigmentverteilung auf der Haut. Der Fachmann hätte zunächst angenommen, dass eine höhere Stabilität nur durch eine erhöhte Emulgatorkonzentration zu erzielen ist. Höhere Emulgatorkonzentrationen wirken für die erfindungsgemäße Zubereitung jedoch kontraproduktiv.

In der erfindungsgemäßen Zubereitung kann das Titandioxid unbeschichtet oder mit Stearinsäure und/oder Aluminiumstearat beschichtet sein. Diese Beschichtung bietet mehrere zusätzliche Vorteile:
**Verbesserte Dispergierbarkeit:** Die Beschichtung mit Stearinsäure verbessert die Dispergierbarkeit von Titandioxid in verschiedenen Medien, insbesondere in Öl- und wasserbasierten Formulierungen. Dies führt zu einer gleichmäßigeren Verteilung der Partikel.

**Reduziertes Weißelverhalten:** Die Stearinsäurebeschichtung kann das Weißelverhalten von Titandioxid weiter reduzieren. Dadurch hinterlassen die kosmetischen Produkte noch weniger sichtbare weiße Rückstände auf der Haut.

**Erhöhte Stabilität:** Die Beschichtung schützt die Titandioxidpartikel weiter vor Agglomeration und Sedimentation, was die Stabilität der Formulierung verbessert und die Wirksamkeit des Produkts weiter erhöht.

**Verbesserte Hautverträglichkeit:** Die Stearinsäurebeschichtung kann die Hautverträglichkeit von Titandioxid weiter erhöhen, indem sie die Reibung zwischen den Partikeln und der Hautoberfläche reduziert. Dies führt zu einem angenehmeren Hautgefühl.

**Erhöhte Hydrophobie:** Die hydrophobe Natur der Stearinsäure verbessert die Wasserabweisung der Titandioxidpartikel. Dies ist besonders vorteilhaft für wasserfeste Sonnenschutzmittel.

**Optimierte UV-Schutzleistung:** Die gleichmäßige Verteilung und Stabilität der beschichteten Titandioxidpartikel tragen zu einer effektiveren UV-Schutzleistung bei, da die Partikel eine gleichmäßigere Schutzschicht auf der Haut bilden.

**Verbesserte Formulierbarkeit:** Durch die verbesserte Dispergierbarkeit und Stabilität lassen sich kosmetische Produkte mit beschichtetem Titandioxid leichter und effizienter formulieren. Dies kann die Herstellungskosten senken und die Produktqualität erhöhen.

**Verringerte Photokatalytische Aktivität:** Die Stearinsäurebeschichtung kann die photokatalytische Aktivität von Titandioxid reduzieren. Dies bedeutet, dass das Risiko der Bildung freier Radikale und damit verbundener Hautschäden verringert wird. Alternativ ist auch ein Coating mit Silica (Siliziumdioxid und/oder Kieselsäure) möglich. Als Zinkoxid können Partikel eingesetzt werden, deren Primärpartikelgröße kleiner 100 nm beträgt. Die hier eingesetzten Zinkoxidpartikel können unbeschichtet sein.

Bevorzugt ist die erfindungsgemäße Zubereitung frei von synthetischen organischen Lichtschutzfiltern, synthetischen Duftstoffen, frei von synthetischen Farbstoffen, frei von Silikonen und Silikonderivaten, frei von mit ionisierender Strahlung bestrahlten Pflanzeninhaltstoffen, frei von genmodifizierten Inhaltstoffen, frei von Paraffinen, frei von Phthalaten, frei von Mineralöl, frei von Mikrokunststoffkugeln, frei von Parabenen. Dabei bedeuten die Worte "frei von" einen Gehalt von 0 Gewichtsprozent, bezogen auf das Gesamtgewicht der Zubereitung. Die Zubereitung kann einen pH-Wert im Bereich 5,0 bis 8, oder im Bereich von 6,0 bis 7,5 aufweisen. Der pH-Wert der Zubereitung kann mit Milchsäure oder Zitronensäure und Natronlauge eingestellt werden.

Die erfindungsmäße Zubereitung kann darüber hinaus w/o- als auch o/w-Emulgatoren enthalten wie z.B. nicht abschließend Glycerylstearatcitrate, Kaliumcetylphosphate, Natriumstearoylglutamat, Cetearylolivat, Sorbitanolivat, Polyglyceryl-6 Stearat (und) Polyglyceryl-6behenat, Polyglyceryl-3-dicitrat/stearat als auch Polyglyceryl-4-olivat/Polyricinoleat, Polyglyceryl-4-diisostearat/Polyhydroxystearat/Sebacat, Glyceryloleat (und) Polyglyceryl-3-polyricinoleat (und) Olea Europaea (Olive) Oil Unsaponifiables.

Ebenso können in der erfindungsgemäßen Zubereitung Wirkstoffe wie z.B. Ubiquinon 10 (Q10), Hyaluronsäuren, Bakuchiol, Retinol, Ferulasäure, Vitamin C, Vitamin E, Pongamol, Resveratrol, Peptide, Proteine, Aminosäuren sowie Pflanzenextrakte enthalten sein.

Zusätzlich kann ein oder mehrere Antioxidans/Antioxidantien enthalten sein. Das eine oder mehrere Antioxidans/Antioxidantien kann/können ausgewählt sein aus Tocopherol und Ascorbylpalmitat. Weiter kann die Zubereitung mindestens ein pflanzliches Öl enthalten. Das mindestens eine pflanzliche Öl kann ausgewählt sein aus der Gruppe bestehend aus z.B. Olivenöl, Sojaöl, Mandelöl, Jojobaöl, Traubenkernöl und Sonnenblumenöl. Solche Öle werden auch als Olea Europaea Fruit Oil, Glycine Soja Oil, Prunus Amygdalus Dulcis Oil, Simmondsia Chinensis Seed Oil, Vitis Vinifera Seed Oil, Pongamia Glabra Seed Oil und Helianthus Annuus Seed Oil bezeichnet. Weiter kann ein Gehalt an natürlicher, biotechnologisch hergestellter Hyaluronsäure 0,01 bis 1 Gew.% betragen. Weiter kann ein Gehalt an Rapsölsterolen 0,01 bis 1 Gew.% betragen. Weiter kann ein Gehalt an Lecithin 0,01 bis 3 Gew.% betragen. Weiter kann ein Gehalt an Polysacchariden 0,1 bis 5 Gew.% betragen.

Coco-Caprylate kann in der Zubereitung in einer Menge von 1 bis 40 Gewichtsprozent, 1 bis 35 Gewichtsprozent oder 1 bis 30 Gewichtsprozent, bezogen auf das Gesamtgewicht der Zubereitung, vorliegen. Alternativ kann anstelle Coco-Caprylat/Caprate eingesetzt werden.

Darüber hinaus können Pigmente für Foundations, Make Ups, BB und CC Cremes als auch Schimmerpigmente für einen besonderen Glow Effekt und zur Auffrischung des Teints enthalten sein. Dies können u.a. folgende Pigmente und Farbstoffe sein: CI 77491, CI 77007, CI 77499 ,CI 77891, CI 75470, CI 77288, CI 77492, CI 77163, Mica, Calcium Aluminium Borosilicate etc.

Die erfindungsgemäße Zubereitung kann ferner bestimmte Formen des Pongamols einschließen, z.B. Pongamia Glabra Seed Oil, z.B. in einer Menge von 0,01-10 Gew.-%, Pongamol, z.B. in einer Menge von 0,01-5 Gew.-%, und/oder Pongamia-Extrakt, z.B. in einer Menge von 0,01-5 Gew.-%.

Auch kann die erfindungsgemäße Zubereitung einen oder mehrere UV-Booster enthalten, z.B. Ferulasäure, z.B. in einer Menge von 0,01-2 Gew.-%, und/oder Ethylferulat, z.B. in einer Menge von 0,01-2 Gew.-%.

Schließlich kann die erfindungsgemäße Zubereitung auch einen oder mehrere Emollienten umfassen, z.B. Caprylic/Capric/Myristic/Stearic Triglyceride, z.B. in einer Menge von 0,1-5 Gew.-%.

Alle Gewichtsprozent-Angaben in der vorliegenden Anmeldung beziehen sich auf die gesamte Zubereitung, sofern nicht anders angegeben.

Die Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, weitere Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte oder organische Lösungsmittel, sofern der Zusatz die geforderten Eigenschaften etwa der Farbe und ggf. mikrobiologischen Stabilität sowie Hautverträglichkeit nicht beeinträchtigt.

Die erfindungsgemäßen Zubereitungen umfassen vorzugsweise nur Naturstoffe, natürliche, naturnahe und/oder naturidentische Rohstoffe. Naturstoffe sind Substanzen pflanzlichen, tierischen und/oder mineralischen Ursprungs sowie deren Gemische und Reaktionsprodukte. Als natürliche, naturnahe und/oder naturidentische Rohstoffe sind Stoffe anzusehen, die aus Naturstoffen nur durch physikalische Mittel gewonnen werden, nur chemisch verändert werden bzw. naturidentisch hergestellt werden.

Erfindungsgemäße Zubereitungen können in verschiedenen Formen vorliegen. So können sie z. B. eine Lösung, eine Emulsion oder Mikroemulsion vom Typ Wasser-in-Öl (W/O), eine multiple Emulsion, beispielsweise vom Typ Wasser-in-Öl-in-Wasser (W/O/W), eine Emulsion vom Typ Öl-in-Wasser (O/W) oder eine Gelemulsion bzw. Hydrodispersionsgel darstellen.

Nachfolgende Ausführungsbeispiele veranschaulichen die erfindungsgemäßen Zubereitungen. Die Angaben sind Gewichtsanteile jeweils bezogen auf das Gesamtgewicht der Zubereitung.

Weitere Merkmale der vorliegenden Erfindung ergeben sich aus der nachfolgenden Beschreibung der Ausführungsbeispiele. Dabei bilden alle beschriebenen Merkmale für sich und in beliebiger Kombination den Gegenstand der Erfindung auch unabhängig von ihrer Zusammensetzung in den einzelnen Ansprüchen oder deren Rückbezügen.

**Darstellung exemplarischer Ausführungsformen der Erfindung SPF 30 und SPF 50 in zertifizierter Naturkosmetikqualität (NATRUE)**

| | | | |
|---|---|---|---|
| ZINCOXID (nano) | 22 | 24 | 15 |
| TITANDIOXID (nano) | 9 | 10 | 8 |
| ALUMINUMSTEARAT | 3 | 3 | 2 |
| ALUMINA | 1 | 1 | 0,79 |
| POLYHYDROXYSTEARINSÄURE | 1,5 | 3 | 1 |
| ISOSTEARINSÄURE | 0,5 | 1,5 | 0,25 |
| POLYGLYCERYL-2-OLEAT | 0,25 | 0,5 | 1 |
| POLYGLYCERYL-2-STEARAT | 0,1 | 0,5 | 1 |
| POLYGLYCERYL-4-DIISOSTEARAT/POLYHYDROXYSTEARAT/SEB ACAT | 3 | 2 | 2,5 |
| MAGNESIUMSULFAT | 0,5 | 0,7 | 0,5 |
| SILICA | 1 | 2 | 0 |
| TOCOPHEROL | 0,5 | 1 | 2 |
| CAPRYLYLGLYCOL | 3 | 2 | 2 |
| Caprylic/Capric/Myristic/Stearic Triglycerid | 1 | 2 | 0 |
| GLYCERYLCAPRYLAT | 2 | 2 | 4 |
| GLYCERIN | 2 | 1 | 4 |
| COCO-CAPRYLAT | 4 | 7 | 20 |
| Caprylic/Capric Triglycerid | 2 | 6 | 5 |
| GLYCIN SOJAÖL | 2 | 1 | 3 |
| HELIANTHUS ANNUUS SEED ÖL | 4 | 1 | 2 |
| BUTYROSPERMUM PARKII BUTTER | 1 | 0,5 | 0 |
| PRUNUS AMYGDALUS DULCIS ÖL | 2 | 1 | 3 |
| SIMMONDSIA CHINENSIS SEED ÖL | 1 | 1 | 2 |
| PERSEA GRATISSIMA ÖL | 2 | 0 | 1 |
| ALOE BARBADENSIS LEAF SAFT | 1 | 3 | 2 |
| Pflanzenextrakt 1 | 1 | 2 | 0,5 |
| Pflanzenextrakt 2 | 5 | 1 | 0 |
| ZITRONENSÄURE | 0,021 | 0,021 | 0,02 |
| Parfum | 0 | 0,36 | 0,36 |
| WASSER ad 100 | ad 100 | ad 100 | ad 100 |

Als Pflanzenextrakt 1 und 2 können beliebige Pflanzenextrakte eingesetzt werden, z.B. Mango- oder Pfirsichextrakt.

Ergänzend sei darauf hingewiesen, dass "aufweisend" keine anderen Elemente oder Schritte ausschließt und "ein" oder "einer" keine Vielzahl ausschließt. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können.

## Patentansprüche

1. Kosmetische Zubereitung, die mindestens einen mineralischen partikulären Lichtschutzfilter, Polyglyceryl-4-diisostearat/Polyhydroxystearat/Sebacat, Polyhydroxystearinsäure, Isostearinsäure, Polyglyceryl-2-oleat und Polyglyceryl-2-stearat umfasst.

2. Kosmetische Zubereitung nach Anspruch 1, wobei der mindestens eine mineralische Lichtschutzfilter ausgewählt ist aus der Gruppe bestehend aus partikulärem Titandioxid, partikulärem Zinkoxid und einer Mischung derselben.

3. Kosmetische Zubereitung nach Anspruch 2, wobei das partikuläre Titandioxid Titandioxid mit einer Partikelgröße in einem Bereich von 2 bis 200 nm umfasst.

4. Kosmetische Zubereitung nach Anspruch 2 oder 3, wobei das partikuläre Titandioxid mit Stearinsäure und/oder Aluminiumstearat beschichtet ist.

5. Kosmetische Zubereitung nach einem der Ansprüche 2 bis 4, wobei das partikuläre Zinkoxid mit einer Partikelgröße in einem Bereich von 2 bis 200 nm umfasst.

6. Kosmetische Zubereitung nach einem der vorangehenden Ansprüche, welche Zubereitung Coco-Caprylate umfasst.

7. Kosmetische Zubereitung nach einem der vorangehenden Ansprüche, welche Zubereitung Coco-Caprylate/Caprate umfasst.

8. Kosmetische Zubereitung nach einem der vorangehenden Ansprüche, wobei die Zubereitung mindestens einen Wirkstoff ausgewählt aus der Gruppe bestehend aus Pongamia-Extrakt, Pongamol, Pongamia Glabra Seed Oil und Kombinationen derselben enthält.

9. Kosmetische Zubereitung nach einem der vorangehenden Ansprüche, wobei die Zubereitung ein oder mehrere Antioxidans/Antioxidantien umfasst.

10. Kosmetische Zubereitung nach Anspruch 8, wobei das eine oder mehrere Antioxidans/Antioxidantien ausgewählt ist/sind aus Tocopherol und Ascorbylpalmitat.

11. Kosmetische Zubereitung nach einem der vorangehenden Ansprüche, wobei die Zubereitung mindestens ein pflanzliches Öl umfasst.

12. Kosmetische Zubereitung nach Anspruch 10, wobei das mindestens eine pflanzliche Öl ausgewählt ist aus der Gruppe bestehend aus Olivenöl, Sojaöl, Mandelöl, Jojobaöl und Sonnenblumenöl.

13. Verwendung der kosmetischen Zubereitung nach einem der vorangehenden Ansprüche zum Schutz vor Sonnenlicht.
